# EUROPEAN PATENT SPECIFICATION

(11) **EP 1 237 539 B1**
(45) Date of publication and mention of the grant of the patent: **19.10.2005**
(21) Application number: 00975872.3
(22) Date of filing: 16.10.2000
(51) Int. Cl.: A61K 9/08, A61K 31/55, A61P 25/28

(54) **ORAL SOLUTION CONTAINING GALANTHAMINE AND A SWEETENING AGENT**
ORALE LÖSUNG ENTHALTEND GALANTHAMINE UND EIN SÜSSUNGSMITTEL
SOLUTION ORALE CONTENANT DE LA GALANTHAMINE ET UN EDULCORANT

(30) Priority: 26.10.1999 EP 99203512
(43) Date of publication of application: 11.09.2002
(73) Proprietor: JANSSEN PHARMACEUTICA N.V., 2340 Beerse (BE)
(72) Inventor: FRANCOIS, Marc, Karel, Jozef Janssen Pharmaceutica, B-2460 Beerse (BE); KEMPEN,Tony,Mathilde,J. Janssen Pharmaceutica N.V., B-2340 Beerse (BE); DE PROOST,Eddy,A,Josée Janssen Pharmaceutica N.V., B-2340 Beerse (BE)
(74) Representative: Quaghebeur, Luc
(86) International application number: PCT/EP2000/010203
(87) International publication number: WO 2001/030318

(56) References cited:
- EP-A- 0 449 247
- EP-A- 0 556 057
- EP-A- 0 653 427
- DATABASE WPI Section Ch, Week 199851 Derwent Publications Ltd., London, GB; Class B05, AN 1998-603235 XP002132712 & JP 10 273435 A (FUJISAWA PHARM CO LTD), 13 October 1998 (1998-10-13)

## Description

The present invention concerns an oral solution comprising galantamine or a pharmaceutically acceptable addition salt thereof; its use and process of preparing the same.

Galantamine (I), a tertiary alkaloid, has been isolated from the bulbs of the Caucasian snowdrops *Galanthus woronowi* (Proskurnina, N. F. and Yakoleva, A. P. 1952, Alkaloids of *Galanthus woronowi.* II. Isolation of a new alkaloid. (In Russian.) Zh. Obschchei Khim. (J. Gen. Chem.) 22, 1899-1902). It has also been isolated from the common snowdrop *Galanthus nivalis* (Boit, 1954).

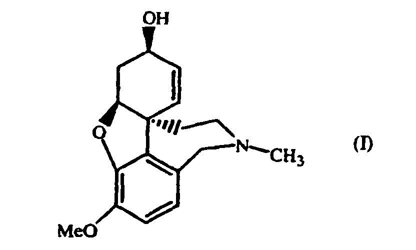

The chemical name of galantamine is [4aS-(4aα, 6β, 8aR*)]-4a, 5, 9, 10, 11, 12-hexahydro-3-methoxy-11-methyl-6*H*-benzofuro[3a, 3, 2-ef][2]benzazepin-6-ol ; both the base compound and its hydrobromide are laevorotatory. Galantamine is a well-known acetylcholinesterase inhibitor which is active at nicotinic receptor sites but not on muscarinic receptor sites. It is capable of passing the blood-brain barrier in humans, and presents no severe side effects in therapeutically effective dosages.

Galantamine has been used extensively as a curare reversal agent in anaesthetic practice in Eastern bloc countries (cf. review by Paskow, 1986) and also experimentally in the West (cf. Bretagne and Valetta, 1965: Wislicki, 1967; Consanitis, 1971).

Galantamine has been marketed by Waldheim (Sanochemia Gruppe) as Nivalin™ in Germany and Austria since the 1970s for indications such as facial neuralgia.

The use of galantamine or an analogue or a pharmaceutically acceptable acid addition salt thereof for the preparation of a medicament for treating Alzheimer's Dementia (AD) and related dementias has been described in EP-0,236,684 (US-4,663,318). This patent generically discloses liquid galantamine dosage forms, more in particular oral suspensions or solutions in aqueous ethanol.

EP 0,449,247 generically discloses solutions or suspensions of galantamine or a pharmaceutically acceptable addition salt thereof in organic or inorganic media, such as oils or water, for the treatment of alcoholism. WO 94/16708 generically discloses the same compositions for the treatment of nicotine dependency. EP 653,427 relates to galantamine derivatives and formulations thereof.

WO 97/26887 describes ocular, oral and parenteral aqueous solutions comprising galantamine or a pharmaceutically acceptable addition salt thereof for the treatment of glaucoma, trisomy or myasthenia gravis. These liquid dosage forms are only generically described.

Oral administration of a liquid galantamine dosage form may offer an attractive way to treat patients suffering from Alzheimer disease and related dementia, vascular dementia, mixed (Alzheimer and vascular) dementia, mild cognitive impairment (MCI), Lewy body disease (LBD), Parkinson disease, schizophrenia, arthritic disorders, chronic fatigue syndrome, facial neuralgia, attention deficit disorders, obstructive sleep apnoea, jet lag, alcohol dependence, nicotine dependence, mania, trisomy, myasthenia gravis, Eaton-Lambert syndrome. The present invention further relates to a method of treating wanm-blooded animals suffering from Alzheimer disease and related dementia, vascular dementia, mixed (Alzheimer and vascular) dementia, mild cognitive impairment (MCI), Lewy body disease (LBD), Parkinson disease, schizophrenia, arthritic disorders, chronic fatigue syndrome, facial neuralgia, attention deficit disorders, obstructive sleep apnoea, jet lag, alcohol dependence, nicotine dependence, mania, trisomy, myasthenia gravis, Eaton-Lambert syndrome because of ease of administration. Solid oral dosage forms such as tablets or capsules are not the most suitable dosage forms to treat said conditions since their administration can be a problem (swallow resistance or difficulties). Oral administration is preferred to parenteral administration because the latter is inconvenient and painful and reduces patient's compliance.

Thus, the present invention concerns an oral solution comprising galantamine or a pharmaceutically acceptable addition salt thereof characterized in that it comprises from 0.005 to 3 % (w/v) of a sweetening agent.

When being dissolved in an aqueous medium, galantamine exhibits a slightly unpleasant taste. Surprisingly, this unpleasant taste can completely be masked by including a sweetening agent from 0.005 to 3 % (w/v; weight based on the total volume of the formulation), preferably from 0.01 to 1% (w/v), more preferably from 0.01 to 0.1 % (w/v) and most preferred is 0.05% (w/v). Consequently no additional flavouring agents are required. Suitable sweetening agents are preferably intense sweeteners, i.e. agents with a high sweetening power when compared to sucrose (e.g. at least 10 times sweeter than sucrose). Suitable intense sweeteners comprise aspartame, saccharin, sodium or potassium or calcium saccharin, acesulfame potassium, sucralose, alitame, cyclamate, neomate, neohesperidine dihydrochalcone or mixtures thereof, thaumatin, palatinit, stevioside and rebaudioside, sodium saccharin being preferred.

Galantamine or a pharmaceutically acceptable addition salt thereof dissolved in an aqueous medium is most stable in weak acid conditions (pH = ± 5), whereas it decomposes in acidic and alkaline medium.

For commercial sale, oral solutions are often filled into glass containers. It is a known phenomenon that glass can leach hydroxy ions, affecting in this way the pH and possibly the stability of its contents. It is general practice to assure a stable pH by including buffering agents in formulations, especially when packed in untreated glass containers. However, including extra excipients in a formulation increases the risk of drug-excipients or excipients-excipients interactions. It also increases the risk of adverse side effects experienced by patients taking the medication. From a commercial point of view, it increases the cost of the end product.

When filled in USP type III amber glass bottles, the pH of the present oral solution proved to remain within the preferred shelf-live specification (pH 4-8), without the incorporation of buffering agents. Thus the bulk liquid carrier of the solution of the present invention is a plain aqueous solution, i.e. a non-buffered aqueous solution.

The term 'bulk liquid carrier' defines the major part of the solution, preferably ranging from about 70 up to about 99 % (w/w; weight based on the total weight of the formulation), more preferably ranging from about 80 up to about 99 % (w/w). The water making up the bulk liquid carrier is preferably purified water or demineralized water, purified water being preferred.

By adding suitable pharmaceutically acceptable acids or bases, the pH of the solution of the present invention can be adjusted to range from 4 to 8, preferably from 4 to 6, more preferably from 5 to 6 and most preferred is 5. Suitable pharmaceutically acceptable acids comprise inorganic acids, such as hydrohalic acids, e.g. hydrochloric or hydrobromic acid, sulfuric, nitric, phosphoric and the like acids, or organic acids, such as, for example, acetic, propanoic, hydroxyacetic, lactic, pyruvic, oxalic, malonic, succinic, maleic, fumaric, malic, tartaric, citric, ascorbic and the like acids.
Appropriate bases comprise organic and inorganic bases, for example ammonium acetate, ammonia, alkali or earth alkaline metal hydroxides, sodium carbonate, sodium hydrogen carbonate, sodium phosphate and the like.

In order to increase the shelf life of the present solution, which is likely to be used repeatedly, the growth of micro-organisms such as bacteria, yeasts and fungi in the formulation may be prevented by adding one or more preservatives. Pharmaceutically acceptable preservatives include quaternary ammonium salts such as lauralkonium chloride, benzalkonium chloride, benzododecinium chloride, cetyl pyridium chloride, cetrimide, domiphen bromide; alcohols such as benzyl alcohol, chlorobutanol, o-cresol, chlorocresol, phenol, phenyl ethyl alcohol, organic acids or salts and derivatives thereof such as benzoic acid, sodium benzoate, sorbic acid, potassium sorbate, parabens such as methyl parahydroxybenzoate or propyl parahydroxybenzoate, aqua conservans; phenylmercuri nitrate,-borate,-acetate; chloorhexidine diacetate,-digluconate. The formulation may also contain anti-oxydants, such as, for example, sodium metabisulfite, sodium bisulfite, sodium sulfite, sodium thiosulfate, ascorbic acid, or complex forming agents such as EDTA, citric acid, tartaric acid, sodium-hexametaphosphate and the like. The concentration of the preservative will range from 0% to 2% (w/w), depending on the actual preservative being used. Preferable preservatives in the composition of the present invention are paraben preservatives, more in particular a mixture of methyl parahydroxybenzoate and propyl parahydroxybenzoate. The concentration of the anti-oxydants generally amounts up to 0.2 % (w/v) and the amount of complex forming agents up to 3 % (w/v).

Although a flavouring agent is not required to mask the unpleasant taste of galantamine in the present solution, one or more flavouring substances may optionally be added to the subject invention to further optimize its palatability. Suitable flavouring substances are fruit flavours such as cherry, raspberry, black currant or strawberry flavour, or stronger flavours, such as Caramel Chocolate flavour, Mint Cool flavour, Fantasy flavour and the like. Combinations of flavours are advantageously used. The total concentration of the flavouring substances may range from 0.01% to 0.5%, preferably from 0.03% to 0.2% and most preferably from 0.05% to 0.1%.

The oral solution of the present invention may also optionally include viscosity regulating agents, for example, alkylcelluloses such as methylcellulose ; hydroxyalkylcelluloses such as hydroxymethylcellulose, hydroxyethylcellulose, hydroxypropylcellulose and hydroxybutylcellulose ; hydroxyalkyl alkylcelluloses such as hydroxyethyl methylcellulose and hydroxypropyl methylcellulose ; carboxyalkylcelluloses such as carboxymethylcellulose ; alkali metal salts of carboxyalkylcelluloses such as sodium carboxymethylcellulose ; carboxyalkylalkylcelluloses such as carboxymethylethylcellulose ; carboxyalkylcellulose esters ; starches ; pectines such as sodium carboxymethylamylopectine ; chitin derivates such as chitosan ; di-, oligo- and polysaccharides such as trehalose, cyclodextrins and derivatives thereof, alginic acid, alkali metal and ammonium salts thereof, carrageenans, galactomannans, tragacanth, agar-agar, gummi arabicum, guar gummi and xanthan gummi ; polyacrylic acids and the salts thereof ; polymethacrylic acids, the salts and esters thereof, methacrylate copolymers ; polyvinylalcohol ; polyvinylpyrrolidone or copolymers thereof ; polyalkylene oxides such as polyethylene oxide and polypropylene oxide and copolymers of ethylene oxide and propylene oxide.

An interesting composition according to the present invention comprises by weight on the total volume of the composition:

| | |
|---|---|
| Galantamine or a pharmaceutically acceptable addition salt thereof | 0.1 to 2 % |
| Preservative(s) | 0 to 2 % |
| Intense sweetener | 0.005 to 3 % |
| Acid or base | q.s.* ad pH 4-8 |
| Purified water | q.s.* ad 100 %. |

| | |
|---|---|
| *q.s. ad = quantum satis ad = as much as needed up to. | |

The present invention also relates to a process of preparing the oral solution of the present invention comprising the steps of:
- mixing galantamine or a pharmaceutically acceptable addition salt thereof, intense sweetener, optionally other pharmaceutically acceptable excipients and bulk liquid carrier until complete dissolution;
- optionally adjusting the pH of the resulting solution to pH 4-8;
- diluting the resulting solution to the desired end-volume with purified water.

The above general route of preparing the oral solution of the present invention may be modified by a person skilled in the art by for instance adding certain ingredients at other stages than indicated above. For example, the intense sweetener can first be dissolved followed by dissolving galantamine.

A further aspect of the present invention concerns the use of the above formulation as a medicine, especially the use for the manufacture of a medicament for treating patients suffering from Alzheimer disease and related dementia, vascular dementia, mixed (Alzheimer and vascular) dementia, mild cognitive impairment (MCI), Lewy body disease (LBD), Parkinson disease, schizophrenia, arthritic disorders, chronic fatigue syndrome, facial neuralgia, attention deficit disorders, obstructive sleep apnoea, jet lag, alcohol dependence, nicotine dependence, mania, trisomy, myasthenia gravis, Eaton-Lambert syndrome. The present invention further relates to a method of treating warm-blooded animals suffering from Alzheimer disease and related dementia, vascular dementia, mixed (Alzheimer and vascular) dementia, mild cognitive impairment (MCI), Lewy body disease (LBD), Parkinson disease, schizophrenia, arthritic disorders, chronic fatigue syndrome, facial neuralgia, attention deficit disorders, obstructive sleep apnoea, jet lag, alcohol dependence, nicotine dependence, mania, trisomy, myasthenia gravis, Eaton-Lambert syndrome by administering to said warm-blooded animals an therapeutically effective amount of the oral solution of the present invention.

The daily required dosage of galantamine or a pharmaceutically acceptable addition salt thereof, the amount per single dose and the frequency of dosing varies with the condition being treated, the severity of said condition, and the patient being treated. The daily dosage may range from 5 to 1000 mg, preferably from 5-45 mg, more preferably from 10-35 mg and most preferred from 15-25 mg.

### Experimental part

### (a) Composition

| | |
|---|---|
| Galantamine hydrobromide | 5.124 mg (4 mg of galantamine base) |
| Methyl parahydroxybenzoate | 1.800 mg |
| Propyl parahydroxybenzoate | 0.200 mg |
| Sodium saccharin dihydrate | 0.500 mg |
| Sodium hydroxide | q.s.* ad pH 4.9-5.1 |
| Purified water | q.s.* ad 1.0 ml. |

| | |
|---|---|
| *q.s. ad = quantum satis ad = as much as needed up to. | |

### (b) Preparation of a 300 l batch

150 l of purified water was transferred into a stainless steel liquid processor and was heated up to 45-50°C, while stirring. Methyl parahydroxybenzoate (0.54 kg) and propyl parahydroxybenzoate (0.06 kg) were added and the resulting mixture was stirred until complete dissolution. 135 l of purified water was added and the whole was stirred until homogeneous and cooled down to 20-30°C. Galantamine hydrobromide was added and the mixture was stirred until complete dissolution. Sodium saccharine dihydrate was added and the whole was mixed until complete dissolution. A 0.1 N aqueous sodium hydroxide solution was added to adjust the pH of the solution to 4.9-5.1. Purified water was added to adjust the total volume to 300 l, while mixing until homogeneous. The final solution was filtered over a 25 µm polypropylene filter.

### (c) Stability of a galantamine hydrobromide aqueous solution as a function of pH

A galantamine hydrobromide 10 mg/ml aqueous solution was stored in acid, neutral and alkaline conditions at 80°C for 1 up to 24 hours. After storage, the solutions were analyzed using HPLC for the presence of degradation products. The table below shows the obtained results.

| Medium | Storage | Degradation compounds |
|---|---|---|
| Acid (1N hydrochloric acid) | 1 hour | 18.4 % |
| | | |
| Neutral (water pH 5.2) | 24 hours | No degradation detected |
| | | |
| Alkaline (1N sodium hydroxide) | 24 hours | 0.2 % |

Galantamine hydrobromide remained stable in the aqueous medium of pH 5.2 while it decomposed in acidic and alkaline medium.

### (d) pH stability study of the present solution when filled in glass bottles

The galantamine hydrobromide solution as described under point (a) was filled into 100 ml USP type III amber glass bottles and stored at different conditions. The pH of the solution was determined after predetermined time intervals. The table below gives an overview of the obtained pH values under different conditions.

| Storage condition | Time (months) | pH |
|---|---|---|
| 4°C | 3 | 5.5 |
| 25°C/60% RH* | 1 | 5.5 |
| | 3 | 5.6 |
| | 6 | 5.7 |
| | 9 | 5.5 |
| | 12 | 5.6 |
| 30°C/≤40% RH | 3 | 5.6 |
| | 6 | 5.7 |
| | 9 | 5.6 |
| | 12 | 5.6 |
| 40°C | 1 | 5.7 |
| | 3 | 5.6 |
| | 6 | 5.6 |
| 50°C | 1 | 5.7 |
| | 3 | 5.6 |
| light | 0.3 days | 5.5 |

| | | |
|---|---|---|
| * RH = Relative Humidity | | |

## Claims

1. An oral solution comprising galantamine or a pharmaceutically acceptable addition salt thereof **characterized in that** it comprises from 0.005 to 3 % (w/v) of a sweetening agent and that the bulk liquid carrier is aqueous.

2. An oral solution according to claim 1 wherein the sweetening agent is an intense sweetener.

3. An oral solution according to claim 1 or 2 wherein the bulk liquid carrier is a non-buffered aqueous solution.

4. An oral solution according to claim 1, 2 or 3 wherein the pharmaceutically acceptable addition salt of galantamine is galantamine hydrobromide.

5. An oral solution according to claim 1 or 2 wherein the concentration of the sweetening agent ranges from 0.01 to 1 % (w/v).

6. An oral solution according to claim 5 wherein the sweetening agent is sodium saccharin dihydrate.

7. An oral solution according to claim 1 wherein the pH of the solution is adjusted to range from 4 to 8.

8. An oral solution according to claim 1 having the following composition:
| | |
|---|---|
| Galantamine hydrobromide | 5.124 mg |
| Methyl parahydroxybenzoate | 1.800 mg |
| Propyl parahydroxybenzoate | 0.200 mg |
| Sodium saccharin dihydrate | 0.500 mg |
| Sodium hydroxide | q.s. ad pH 4.9-5.1 |
| Purified water | q.s. ad 1.0 ml. |

9. An oral solution according to claim 1 for use as a medicine.

10. Use of an oral solution as defined in claim 1 for the manufacture of a medicament for the treatment of patients suffering from Alzheimer disease and related dementia, vascular dementia, mixed (Alzheimer and vascular) dementia, mild cognitive impairment (MCI), Lewy body disease (LBD), Parkinson disease, schizophrenia, arthritic disorders, chronic fatigue syndrome, facial neuralgia, attention deficit disorders, obstructive sleep apnoea, jet lag, alcohol dependence, nicotine dependence, mania, trisomy, myasthenia gravis, Eaton-Lambert syndrome.

11. A process of preparing an oral solution as defined in claim 1 comprising the following steps:
- mixing galantamine or a pharmaceutically acceptable addition salt thereof, intense sweetener, optionally other pharmaceutically acceptable excipients and bulk liquid carrier until complete dissolution;
- optionally adjusting the pH of the resulting solution to pH 4-8;
- diluting the resulting solution to the desired end-volume with purified water.

## Patentansprüche

1. Orale Lösung, enthaltend Galantamin oder ein pharmazeutisch unbedenkliches Additionssalz davon, **dadurch gekennzeichnet, daß** sie 0,005 bis 3 % (w/v) eines Süßstoffs enthält und der den Hauptteil ausmachende flüssige Träger wäßrig ist.

2. Orale Lösung nach Anspruch 1, wobei der Süßstoff ein intensiver Süßstoff ist.

3. Orale Lösung nach Anspruch 1 oder 2, wobei der den Hauptteil ausmachende flüssige Träger nicht gepuffert ist.

4. Orale Lösung nach Anspruch 1, 2 oder 3, wobei es sich bei dem pharmazeutischen unbedenklichen Additionssalz von Galantamin um Galantaminhydrobromid handelt.

5. Orale Lösung nach Anspruch 1 oder 2, wobei die Konzentration an Süßstoff im Bereich von 0,01 bis 1% (w/v) liegt.

6. Orale Lösung nach Anspruch 5, wobei es sich bei dem Süßstoff um Natriumsaccharin-dihydrat handelt.

7. Orale Lösung nach Anspruch 1, wobei der pH-Wert der Lösung auf den Bereich von 4 bis 8 eingestellt ist.

8. Orale Lösung nach Anspruch 1 mit der folgenden Zusammensetzung:
| | |
|---|---|
| Galantaminhydrobromid | 5,124 mg |
| Parahydroxybenzoesäuremethylester | 1,800 mg |
| Parahydroxybenzoesäurepropylester | 0,200 mg |
| Natriumsaccharin-dihydrat | 0,500 mg |
| Natriumhydroxid | q.s. ad |
| | pH 4,9-5,1 |
| Gereinigtes Wasser | q.s. ad 1,0 ml. |

9. Orale Lösung nach Anspruch 1 zur Verwendung als Medizin.

10. Verwendung einer wie in Anspruch 1 definierten oralen Lösung zur Herstellung eines Medikaments zur Behandlung von Patienten, die an Alzheimer-Krankheit oder einer verwandten Demenz, vaskulärer Demenz, gemischter (Alzheimer und vaskulärer) Demenz, Mild Cognitive Impairment (MCI), Lewy-Korpuskel-Krankheit (Lewy Body Disease, LBD), Parkinson-Krankheit, Schizophrenie, arthritischen Erkrankungen, chronischem Erschöpfungssyndrom (Chronic Fatigue Syndrom, CFS), Trigeminusneuralgie, Aufmerksamkeitsstörungen, obstruktiver Schlafapnoe, Jetlag, Alkoholabhängigkeit, Nikotinabhängigkeit, Manie, Trisomie, Myasthenia gravis, Lambert-Eaton-Syndrom leiden.

11. Verfahren zur Herstellung einer wie in Anspruch 1 definierten oralen Lösung, welches die folgenden Schritte umfaßt:
- das Mischen von Galantamin oder einem pharmazeutisch unbedenklichen Additionssalz davon, einem intensiven Süßstoff, gegebenenfalls anderen pharmazeutisch unbedenklichen Hilfsstoffen und dem den Hauptteil ausmachenden flüssigen Träger bis zur vollständigen Auflösung;
- gegebenenfalls Einstellen des pH-Wertes der so erhaltenen Lösung auf pH 4-8;
- Verdünnen der so erhaltenen Lösung mit gereinigtem Wasser auf das gewünschte Endvolumen.

## Revendications

1. Solution orale comprenant de la galantamine ou un sel d'addition pharmaceutiquement acceptable de ce composé, **caractérisée en ce qu'**elle comprend de 0,005 à 3% (en poids/volume) d'un agent édulcorant et **en ce que** le véhicule liquide en vrac est aqueux.

2. Solution orale selon la revendication 1 dans laquelle l'agent édulcorant est un édulcorant intense.

3. Solution orale selon la revendication 1 ou 2 dans laquelle le véhicule liquide en vrac est non tamponné.

4. Solution orale selon la revendication 1, 2 ou 3 dans laquelle le sel d'addition pharmaceutiquement acceptable de galantamine est le bromhydrate de galantamine.

5. Solution orale selon la revendication 1 ou 2 dans laquelle la concentration de l'agent édulcorant s'échelonne de 0,01 à 1% (en poids/volume).

6. Solution orale selon la revendication 5 dans laquelle l'agent édulcorant est la saccharine sodique dihydratée.

7. Solution orale selon la revendication 1 dans laquelle le pH de la solution est ajusté dans un domaine de 4 à 8.

8. Solution orale selon la revendication 1 dont la composition est la suivante :
| | |
|---|---|
| bromhydrate de galantamine | 5,124 mg |
| para-hydroxybenzoate de méthyle | 1,800 mg |
| para-hydroxybenzoate de propyle | 0,200 mg |
| saccharine sodique dihydratée | 0,500 mg |
| hydroxyde de sodium | q.s.p. pH 4,9-5,1 |
| eau purifiée | q.s.p. 1,0 ml. |

9. Solution orale selon la revendication 1 à utiliser comme médicament.

10. Utilisation d'une solution orale telle que définie dans la revendication 1 pour la fabrication d'un médicament destiné au traitement de patients souffrant de la maladie d'Alzheimer et de démence apparentée, de démence vasculaire, de mélange de démences (Alzheimer et vasculaire), de déficit cognitif léger (MCI), d'une maladie à corps de Lewy (LBD), de la maladie de Parkinson, de schizophrénie, d'affections arthritiques, de syndrome de fatigue chronique, de névralgie faciale, de troubles déficitaires de l'attention, d'apnée du sommeil d'origine obstructive, de décalage horaire, de dépendance à l'alcool, de dépendance à la nicotine, de manies, de trisomie, de myasthénie grave, de syndrome d'Eaton-Lambert.

11. Procédé de préparation d'une solution orale telle que définie dans la revendication 1 comprenant les étapes suivantes :
- mélanger de la galantamine ou un sel d'addition pharmaceutiquement acceptable de ce composé, un édulcorant intense, éventuellement d'autres excipients pharmaceutiquement acceptables et un véhicule liquide en vrac jusqu'à dissolution complète ;
- ajuster éventuellement le pH de la solution obtenue à pH 4-8 ;
- diluer la solution obtenue jusqu'au volume final souhaité avec de l'eau purifiée.
